(19) Europäisches Patentamt
European Patent Office
Office européen des brevets

(11) Veröffentlichungsnummer: **0 655 242 A1**

(12) # EUROPÄISCHE PATENTANMELDUNG

(21) Anmeldenummer: **94116789.2**

(51) Int. Cl.$^6$: **A61K 31/37**

(22) Anmeldetag: **25.10.94**

(30) Priorität: **08.11.93 DE 4337906**

(43) Veröffentlichungstag der Anmeldung:
**31.05.95 Patentblatt 95/22**

(84) Benannte Vertragsstaaten:
**AT BE CH DE DK ES FR GB IT LI NL SE**

(71) Anmelder: **CASSELLA Aktiengesellschaft**
**Hanauer Landstrasse 526**
**D-60386 Frankfurt (DE)**

(72) Erfinder: **Schönafinger, Karl, Dr.**
**Holunderweg 8**
**D-63755 Alzenau (DE)**
Erfinder: **Klemm, Peter, Dr.**
**Buchenweg 28**
**D-55128 Bretzenheim (DE)**
Erfinder: **Ostrowski, Jörg, Dr.**

**verstorben (DE)**

(74) Vertreter: **Muley, Ralf, Dr.**
**Cassella AG,**
**Patentabteilung,**
**Hanauer Landstrasse 526**
**D-60386 Frankfurt (DE)**

(54) **Verwendung von Cumarinderivaten bei NO-induzierten Krankheiten.**

(57) Die vorliegende Erfindung betrifft somit die Verwendung von Cumarinen der allgemeinen Formel I

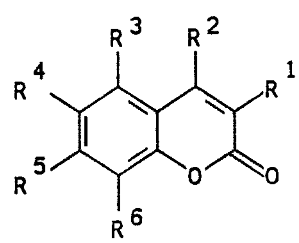

(I)

worin
$R^1$ und $R^6$ wie in den Ansprüchen angegeben definiert sind, zur Bekämpfung und Vorbeugung von Krankheiten, die durch einen erhöhten NO-Spiegel entstehen

EP 0 655 242 A1

Die vorliegende Erfindung betrifft die Verwendung von Cumarinderivaten zur Bekämpfung und Vorbeugung von Krankheiten, die durch einen erhöhten NO-Spiegel entstehen.

Stickstoffmonoxid (NO) spielt in verschiedensten physiologischen Prozessen eine bedeutende Rolle (siehe beispielsweise Spektrum der wissenschaft, Juli 1992, Seite 72). So ist es beispielsweise in der Regulation der Plättchenfunktion und des Blutdrucks, in die Immunabwehr, in cerebrale Funktionen, wie Lern- und Gedächtnisvorgänge, und in Entzündungsprozesse involviert.

Endogenes Stickstoffmonoxid wird aus Arginin mit Hilfe von NO-Synthasen gebildet. Dabei kann man zumindest zwei solcher Enzyme unterscheiden. Erstens eine constitutive $Ca^{2+}$/Calmodulin-abhängige NO-Synthase, die beispielsweise im Endothel und im Gehirn vorkommt und dort auf bestimmte Stimuli hin NO freisetzt und zur Regulation des Blutdrucks und der Plättchenfunktion sowie von cerebralen Funktionen dient. Zweitens eine induzierbare $Ca^{2+}$-unabhängige NO-Synthase, die in einer Vielzahl von Zellen, wie beispielsweise in der glatten Muskulatur, in Makrophagen oder Hepatocyten vorkommt. Letztere kann durch Endotoxin und durch Cytokine induziert werden und setzt dann über lange Zeit große Mengen an NO frei.

Dieses NO ist cytotoxisch für Tumorzellen und für Mikroorganismen und außerdem verantwortlich für schwerwiegende Erkrankungen. In diesem Zusammenhang seien pathologischer Blutdruckabfall beispielsweise beim septischen oder hämorrhagischen Schock, Gewebeschäden und entzündliche Prozesse genannt. Durch die induzierbare NO-Synthase erzeugtes NO spielt aber auch eine wichtige Rolle bei Entstehung und Verlauf von Typ-1-Diabetis und bei der Atherosklerose. Außerdem kann eine hohe Konzentration von NO durch Desaminierung von Cytosin zu DNA-Schäden führen.

Aus der WO 93/13055 ist bereits bekannt, daß bestimmte, vom Arginin abgeleitete Verbindungen Inhibitoren der induzierbaren NO-Synthase sind.

Cumarine sind pharmakologisch wirksame Verbindungen, deren gefäßerweiternde Wirkung speziell auf die Coronararterien lange bekannt und beispielsweise in DE-C 1 210 883, DE-C 1 246 754, DE-C 1 259 349, DE-A 1 668 877, DE-A 1 962 154 oder DE-A 2 247 691 beschrieben ist. Darüberhinaus ist in European Journal of Pharmacology 210 (1992), 107-113, die Wirksamkeit des Cumarinderivats Chloricromen gegen einen endotoxin-induzierten Schock bei Ratten beschrieben.

Es wurde nun überraschenderweise gefunden, daß Cumarine die NO-Freisetzung hemmen und somit hervorragend zur Vorbeugung und Bekämpfung von Krankheiten geeignet sind, die aus einem zu hohen systemischen NO-Spiegel oder zu hohen lokalen NO-Konzentration resultieren.

Die vorliegende Erfindung betrifft somit die Verwendung von Cumarinen der allgemeinen Formel I

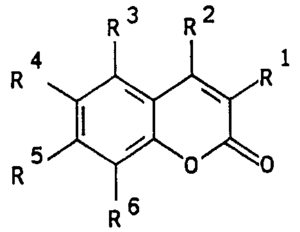

(I)

worin

**$R^1$** Wasserstoff; Halogen; $(C_2\text{-}C_6)$-Alkenyl; $(C_7\text{-}C_{10})$-Aralkyl; Phenyl, das gegebenenfalls substituiert ist durch $(C_1\text{-}C_4)$-Alkyl, $(C_1\text{-}C_4)$-Alkoxy, Halogen, Nitro oder Amino; $(C_1\text{-}C_6)$-Alkyl, Hydroxy-$(C_1\text{-}C_6)$-alkyl oder Dihydroxy-$(C_1\text{-}C_6)$-alkyl, die gegebenenfalls durch $(C_1\text{-}C_4)$-Alkoxy, $(C_1\text{-}C_4)$-Alkoxycarbonyl, $(C_1\text{-}C_4)$-Alkylcarbonyl, Amino oder Aminocarbonyl, die auch N- oder Di-N-substituiert sein können,

$-N^{\oplus}R^7R^8R^9Y^{\ominus}$, 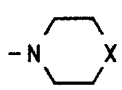 , 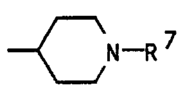 ,

Pyridyl oder Di-$(C_1\text{-}C_4)$-alkylamino-$(C_1\text{-}C_4)$-alkylthio substituiert sind; $-CH(CH_2\text{-}NR^7R^8)_2$; oder Di-$(C_1\text{-}C_4)$-alkylamino-$(C_1\text{-}C_4)$-alkylthio bedeutet;

**$R^2$** $(C_1\text{-}C_6)$-Alkyl, das gegebenenfalls substituiert ist durch

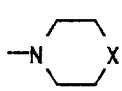

oder Amino oder Aminocarbonyl, die auch N-oder Di-N-substituiert sein können; ($C_2$-$C_6$)-Alkenyl; ($C_6$-$C_{10}$)-Aryl; oder Pyridyl bedeutet;
**$R^3$ und $R^4$** unabhängig voneinander Wasserstoff; Hydroxy; ($C_1$-$C_4$)-Alkoxy, Hydroxy-($C_1$-$C_4$)-alkoxy oder Dihydroxy-($C_1$-$C_4$)-alkoxy, die gegebenenfalls durch ($C_1$-$C_4$)-Alkoxycarbonyl, Amino oder Aminocarbonyl, die auch N- oder Di-N substituiert sein können, oder

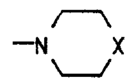

substituiert sind; ($C_2$-$C_6$)-Alkenyloxy; Amino-($C_1$-$C_4$)-alkyl, das auch N- oder Di-N substituiert sein kann;

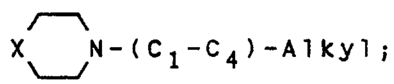

Halogen oder Nitro bedeuten;
**$R^5$** wie $R^3$ definiert ist und zusätzlich auch $CF_3$; $OR^7$; ($C_7$-$C_{10}$)-Aralkoxy; ($C_1$-$C_4$)-Alkoxy, das durch ein oder zwei Hydroxygruppen oder

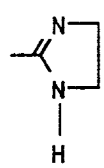

substituiert sein kann; ($C_1$-$C_6$)-Alkoxy, das durch -$COOR^7$ oder -$NR^7R^8$ substituiert ist; ($C_1$-$C_4$)-Alkylcarbonyl; ($C_2$-$C_6$)-Alkinyl; ($C_1$-$C_4$)-Alkoxycarbonyloxy; $NH_2$; oder $NHR^{10}$ bedeutet;
**$R^6$** wie $R^5$ definiert ist aber nicht für Chlor stehen kann, **$R^7$** Wasserstoff; ($C_1$-$C_6$)-Alkyl, Hydroxy-($C_1$-$C_6$)-alkyl oder Dihydroxy-($C_1$-$C_6$)-alkyl, die gegebenenfalls durch Amino, Di-($C_1$-$C_4$)-Alkylamino, ($C_1$-$C_4$)-Alkoxy, Cyan,

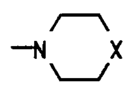

oder einen Rest $R^{10}$ substituiert sind; ($C_2$-$C_6$)-Alkenyl; oder ($C_7$-$C_{10}$)-Aralkyl bedeutet;
**$R^8$** Wasserstoff; ($C_1$-$C_6$)-Alkyl; ($C_1$-$C_4$)-Alkoxy-($C_1$-$C_6$)-alkyl; oder ($C_7$-$C_{10}$)-Aralkyl bedeutet;
**$R^9$** ($C_1$-$C_4$)-Alkyl bedeutet;
**$R^{10}$** Aminocarbonyl; N-($C_1$-$C_4$)-Alkylaminocarbonyl; Di-N-($C_1$-$C_4$)-Alkylaminocarbonyl; N-($C_6$-$C_{10}$)-Arylaminocarbonyl;

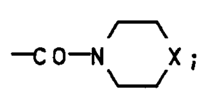

($C_1$-$C_4$)-Alkylcarbonyl; ($C_6$-$C_{10}$)-Arylcarbonyl; Di-N-($C_1$-$C_4$)-Alkylaminomethylcarbonyl; -$SO_2$($C_1$-$C_4$)-Alkyl oder -$SO_2$($C_6$-$C_{10}$)-Aryl bedeutet;
**X** eine Einfachbindung; O; $CH_2$; S; NH; N($C_1$-$C_4$)-Alkyl; N($C_6$-$C_{10}$)-Aryl; oder N(Hydroxy-($C_1$-$C_6$)-alkyl) bedeutet; und
**Y** Cl; Br; I; TosO; $HSO_4$ oder $CH_3COO$ bedeutet, sowie ihrer pharmakologisch annehmbaren Säureadditionsverbindungen zur Bekämpfung und Vorbeugung von Krankheiten, die durch einen erhöhten NO-Spiegel entstehen.

($C_1$-$C_4$)-Alkylgruppen können geradkettig oder verzweigt sein und bedeuten beispielsweise Methyl, Ethyl, n-Propyl, i-Propyl, n-Butyl, i-Butyl, sek-Butyl oder tert.Butyl. Gleiches gilt für ($C_1$-$C_6$)-Gruppen, die

zusätzlich beispielsweise noch n-Pentyl oder n-Hexyl bedeuten können. Für Alkoxy-, Alkylcarbonyl-, Alkylamino- oder Alkylthiogruppen gilt jeweils entsprechendes.

($C_2$-$C_6$)-Alkenyl- und -alkinylgruppen können ebenfalls geradkettig oder verzweigt sein. Alkenylgruppen sind beispielsweise Vinyl und Allyl. Eine Alkinylgruppe ist beispielsweise Ethinyl.

($C_6$-$C_{10}$)-Aralkyl ist insbesondere Benzyl und Phenethyl. ($C_6$-$C_{10}$)-Aryl ist insbesondere Phenyl.

Halogen kann für Fluor, Chlor, Brom oder Iod stehen, wobei Chlor und Brom bevorzugt sind.

Amino oder Aminocarbonyl, die auch N- und Di-N substituiert sein können, haben insbesondere die Formeln -$NR^7R^8$ bzw. -$CONR^7R^8$, wobei $R^7$ und $R^8$ wie oben angegeben definiert sind.

Unter die Formel

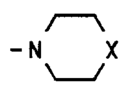

fallen beispielsweise der Pyrrolidinyl-, der Morpholino-, der 1,4-Thiazinyl-, der Piperazinyl- und der Piperidinyl-Rest.

$R^1$ bedeutet bevorzugt ($C_1$-$C_6$)-Alkyl, Di-($C_1$-$C_4$)-alkylamino-($C_1$-$C_6$)-alkyl und 3-Di-($C_1$-$C_4$)-Alkylamino-2-hydroxypropyl. Besonders bevorzugt ist der Diethylaminoethylrest.

$R^2$ bedeutet bevorzugt ($C_1$-$C_6$)-Alkyl oder Phenyl. Besonders bevorzugt ist Methyl.

$R^3$ und $R^4$ bedeutet unabhängig voneinander bevorzugt ($C_1$-$C_4$)-Alkoxy und durch -$NR^7R^8$ substituiertes Ethoxy. Besonders bevorzugt für $R^3$ sind Methoxy und Wasserstoff und $R^4$ Wasserstoff.

$R^5$ bedeutet bevorzugt Hydroxy, Methoxy, ($C_1$-$C_4$)-Alkoxy-carbonylmethoxy, Aminocarbonylmethoxy, Di-($C_1$-$C_4$)-alkylamino-($C_1$-$C_4$)-alkoxy und Amino-($C_1$-$C_4$)-alkoxy.

$R^6$ bedeutet bevorzugt Wasserstoff, Hydroxy, ($C_1$-$C_4$)-Alkoxy, Nitro und Di-($C_1$-$C_4$)-alkylamino-($C_1$-$C_4$)-alkoxy.

Die Verbindungen der allgemeinen Formel I sind bekannt und können nach bekannten Methoden hergestellt werden (siehe beispielsweise Elderfield, Heterocyclic compounds Vol.2, 173-216, (1951) und oben zitierte Patentliteratur), beispielsweise nach der Pechmann'schen Cumarinsynthese.

Erfindungsgemäße Verbindungen der allgemeinen Formel I, die eine basische Gruppe enthalten, können mit anorganischen oder organischen Säuren Salze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Krankheiten, die durch einen erhöhten NO-Spiegel entstehen und die somit erfindungsgemäß mit den Verbindungen der allgemeinen Formel I behandelt werden können bzw. denen mit diesen vorgebeugt werden kann, sind insbesondere pathologische Blutdruckabfälle, wie sie beim septischen oder hämorrhagischen Schock, bei der Tumortherapie mit Cytokinen oder bei der Leberzirrhose auftreten. Des weiteren entzündliche Erkrankungen, wie rheumatoide Arthritis und insbesondere Colitis ulcerosa, sowie Insulinabhängige Diabetes mellitus und Transplantat Abstoßungsreaktionen.

Aber auch die folgenden Erkrankungen stehen im Zusammenhang mit einer gesteigerten Produktion von Stickstoffmonoxid und können erfindungsgemäß behandelt bzw. vorgebeugt werden. Im Bereich Herz-Kreislauf sind dies Arteriosklerose, postischämische Gewebeschäden, Reperfusionsschäden, Mykarditis auf der Grundlage einer Coxsackie-Virus-Infektion und Kardiomyopathie; im Bereich Zentrales Nervensystem Neuritisformen, Enzephalomyelitiden, virale neurodegenerative Erkrankungen, Morbus Alzheimer, Hyperalgesie, Epilepsie und Migräne; im Bereich Niere akutes Nierenversagen sowie Glomerulonephritis.

Außerdem sind auch Behandlungen im Bereich des Magens und des Uterus/Plazenta sowie eine Beeinflussung der Motilität der Spermien denkbar.

Die Hemmung der NO-Freisetzung durch die Verbindungen der allgemeinen Formel I kann durch NO-Messung mittels Oxyhämoglobin auf Makrophagen wie folgt bestimmt werden:

Mäusemakrophagen (MM) des Stammes RAW 264.7 werden in Petrischalen (10 cm Durchmesser) angezüchtet. Als Nährmedium wird DMEM (Fa. Sigma Nr. D 5405) konditioniert mit 4 mM l-Glutamin, 3,5 g D-Glukose/l, 50 U/50 µg/ml Pen/Strep und 10 % FKS) eingesetzt unter 10 % $CO_2$, 95 % Luftfeuchtigkeit, 37 °C. Die subkonfluenten Zellen werden mit dem Zellschaber abgekratzt, in Medium aufgenommen, die

Zellzahl bestimmt (Zählung in Trypanblaulösung 0,2 %) und in 24 Napfplatten (0,5 x $10^6$ Zellen/ml, 1 ml/Napf) ausgesät.

Ca. 18 Stunden nach Aussaat werden die gut adherierten Mausmakrophagen mit LPS (Lipopolysaccharid von E. coli Serotyp OIIIB4, Fa. Sigma Nr. L 2630) und (γIFN, Maus rekombinant, Fa. Boehringer Mannheim Hr. 1276905) stimuliert. Dazu wird das Kulturmedium von den Zellen abgesaugt und 1 ml/Napf (24 Napfplatte) Inkubationsmedium (MEM; ohne Phenol (Biochrom F0385)) konditioniert mit 4mM Glutamin, 3,5 g Glukose/l, 3,7 g NaHCO3/l, 110 mg Napyruvat/l, 50 U/50 µg/ml Pen/Strep und 5 % FKS) aufgegeben. ProNapf werden 1 µl LPS und 1 µl γIFN pipettiert.

Daraus ergeben sich folgende Konzentrationen der Stimulatoren:

140 ng LPS/ml + 5 U γIFN/ml

Während der Stimulation über 4 Stunden wird die Neusynthese des NO-produzierenden Enzyms NO-Synthase (iNOS) induziert. Nach diesem Inkubationszeitraum wird das Inkubationsmedium abgesaugt und jeder Napf zweimal mit frischem Inkubationsmedium gespült. Versuchsmedium (bestehend aus dem Inkubationsmedium mit Zusatz von SOD 30 U/ml, Katalase 290 U/ml, Indomethacin 3,5 µg/ml, OxyHb ca. 8 µM und Testsubstanz (100,500 µM)) wird in die Näpfe gegeben (1 ml/Napf bei 24 Napfplatte). Als Kontrolle wird das konditionierte Versuchsmedium nur mit dem Lösungsmittel der Testsubstanz versetzt. Die mit Versuchsmedium beschickten Platten werden 120 Minuten im Brutschrank bei 37°C, 10 % $CO_2$ und 95 % Luftfeuchtigkeit inkubiert. Nach der 120-minütigen Inkubation wird das Versuchsmedium in Kunststoff-Halbmikroküvetten pipettiert und die Extinktionsdifferenz 576-592 nm am DU 70 gemessen.

Je 2 Näpfe werden mit einer Testsubstanz beschickt (n = 2), auf jeder Platte wird eine Kontrolle unstimuliert, eine Stimulationskontrolle und eine Positiv-Kontrolle (Nitro-L-Arginin, 500 µM) vergleichend zu den Testsubstanzen untersucht. Als Maßangabe für die Testsubstanzen wird die Extinktionsdifferenz 576-592 nm prozentual zur Stimulationskontrolle verglichen. Von dem mit Testsubstanz versetzten Versuchsmedium wird zum Zeitpunkt 0 die Extinktionsdifferenz 576-592 nm gemessen sowie der pH-Wert und die Osmolalität bestimmt.

Nach dem Abpipettieren des Versuchsmediums wird die Cytotoxizität der Testsubstanz geprüft (Zeitviabilität, DNS-Synthese).

Mittels dieser Methode konnte festgestellt werden, daß Carbocromen ($R^1$ = Diethylaminoethyl, $R^2$ = Methyl, $R^5$ = Ethoxycarbonylmethoxy, $R^3$, $R^4$, $R^6$ = Wasserstoff, Hydrochlorid) in einer Menge von 250µM die NO-Freisetzung aus Mausmakrophagen sowohl in der Induktionsphase als auch nach Stimulation mit LPS/γ-IFN zu 85 bis 100 % hemmt.

Die Wirkung der Verbindungen der allgemeinen Formel I auf den Blutdruck kann in folgendem in vivo-Versuch bestimmt werden:
An männlichen Sprague-Dawley Ratten (280 bis 440 g) erfolgt zunächst eine Vollnarkose durch intraperitoneale Injektion von Natrium-Pentobarbital (60 bis 100 mg/kg). Nach Einsetzen der Narkose wird eine Tracheotomie durchgeführt und die Ratten werden mit Raumluft (10 bis 13 ml $kg^{-1}$) bei 54 Atemzügen/min beatmet. Anschließend werden die peripheren Venen (V. jugularis, V. femorales) freigelegt und Katheter für die intravenöse Gabe der Testsubstanzen sowie für Blutentnahmen eingebunden. Ein Katheter wird in die linke A. carotis eingebunden und mit einem Druckaufnehmer (Typ P23 Dd, Statham, Hato Ray, Puerto Rico) verbunden. Die Registierung des systolischen (BPs) und diastolischen (BPd) = Blutdrucks wird über einen Brush Mark 220 Polygraph realisiert.

Am Ende eines Versuchs (nach 90 min) werden die Ratten entblutet und das Plasma gewonnen. Unter Zuhilfenahme der Griess Reaktion wird die Nitrit ($NO_2^-$)Plasmakonzentration bestimmt.

Zur Durchführung dieses Versuchs mit Carbocromen wurden vier Gruppen mit je vier Individuen gebildet. Gruppe 1 erhielt Kochsalz (0,3 ml), Gruppe 2 wurden Carbocromen in einer Konzentration von 10 mg/kg verabreicht, Gruppe 3 erhielt eine Bolusinjektion Lipopolysaccharid (LPS) (E.coli, Serotyp 127:B08, TCA-Extract, Sigma Deisenhofen) in einer Konzentration von 15 mg/kg, und Gruppe 4 erhielt Carbocromen und LPS, wobei das Carbocromen 10 Minuten vor dem LPS verabreicht wurde. Alle Substanzen wurden intravenos gegeben.

Die Ergebnisse sind in folgender Tabelle angegeben:

| Gruppe | | Blutdruck (mmHg) Zeit (min) 0 | 15 | 30 | 60 | 90 | $NO_2$-Plasma-konzentration (nmol/ml) |
|---|---|---|---|---|---|---|---|
| 1 | BPs | 125±5 | 125±5 | 125±3 | 130±6 | 130±3 | 4,5 |
| | BPd | 95±6 | 90±6 | 85±4 | 85±5 | 85±5 | |
| 2 | BPs | 125±5 | 125±5 | 125±3 | 130±6 | 130±3 | 4,5 |
| | BPd | 95±6 | 90±6 | 85±4 | 85±5 | 85±5 | |
| 3 | BPs | 125±6 | 55±6 | 75±5 | 70±4 | 50±6 | 9,3 |
| | BPs | 95±7 | 30±5 | 45±6 | 35±5 | 25±5 | |
| 4 | BPs | 125±6 | 120±6 | 120±5 | 110±4 | 110±6 | 4,9 |
| | BPd | 90±5 | 75±5 | 70±6 | 70±5 | 70±6 | |

Die Verbindungen der allgemeinen Formel I und ihre pharmakologisch annehmbaren Säureadditionssalze können am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Säureadditionssalzes davon, neben üblichen pharmazeutisch einwandfreien Trägerund Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z.B. Propranolol, Pindolol, Metoprolol; Vasodilatatoren, wie z.B. Carbocromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Ramipril, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die folgenden Beispiele zeigen Verbindungen der allgemeinen Formel I, die erfindungsgemäß eingesetzt werden können:

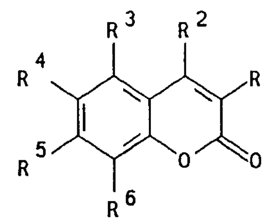

| Beispiel | $R^1$ | $R^2$ | $R^3$ | $R^4$ | $R^5$ | $R^6$ | | Fp(°C) |
|---|---|---|---|---|---|---|---|---|
| 1 | $-CH_2CH_2NEt_2$ | $CH_3$ | H | H | $-OCH_2COOEt$ | H | ·HCl | 156 |
| 2 | $-CH_2CH_2-N$ 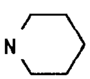 | $CH_3$ | H | H | $-OCH_2COOEt$ | H | ·HCl | 209 |
| 3 | $-CH_2CH_2NEt_2$ | Ph | H | H | $-OCH_2COOEt$ | H | ·HCl | 160 |
| 4 | $-CH_2CH_2-N$ 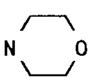 | $CH_3$ | H | H | $-OCH_2COOEt$ | H | ·HCl | 205 |
| 5 | $-CH_2CH_2-N$ 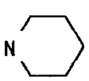 | $CH_3$ | H | H | $-OCH_2CH=CH_2$ | H | ·HCl | 256 |

| Bei-spiel | $R^1$ | $R^2$ | $R^3$ | $R^4$ | $R^5$ | $R^6$ | | Fp($^oC$) |
|---|---|---|---|---|---|---|---|---|
| 6 | $-CH_2CH_2NEt_2$ | $CH_3$ | H | H | -OEt | H | ·HCl | 222 |
| 7 | $-CH_2CH_2NEt_2$ | $CH_3$ | H | H | $-OC_4H_9$ | H | ·HCl | 287 |
| 8 | $-(CH_2)_3NMe_2$ | $CH_3$ | H | H | -OH | H | ·HCl | 233 |
| 9 | $-CH_2CH_2-$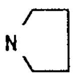 | $CH_3$ | H | H | -OH | H | ·HCl | 261 |
| 10 | -Ph | $CH_3$ | H | H | $-OCH_2CH_2NEt_2$ | H | | 47-50 |
| 11 | $-C_4H_9$ | $CH_3$ | H | H | $-OCH_2CH_2NEt_2$ | H | | 48 |
| 12 | $-CH_2CO_2Et$ | $CH_3$ | OH | H | -OH | H | | 288 |
| 13 | $-C_4H_9$ | $CH_3$ | H | H | -OH | $CH_2NMe_2$ | ·HCl | 205 |
| 14 | -Et | Ph | H | H | $-OCH_2CO_2oPr$ | H | | 125 |
| 15 | $-CH_2Ph$ | $CH_3$ | H | H | $-OCH_2CO_2tert.Bu$ | H | | 126 |
| 16 | $-CH(CH_2NEt)_2$ | $CH_3$ | H | H | $-OCH_2CO_2Et$ | H | ·HCl | 176 |
| 17 | $-CH_2CH_2-$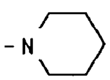 | Ph | H | H | $-O(CH_2)_3CH_3$ | H | | 242 |

| Beispiel | $R^1$ | $R^2$ | $R^3$ | $R^4$ | $R^5$ | $R^6$ | | Fp(°C) |
|---|---|---|---|---|---|---|---|---|
| 18 | $-CH_2CH_2NEt_2$ | $CH_3$ | H | H | $-OCH_2Ph$ | H | | 92 |
| 19 | $-CH_2CH_2NEt_2$ | $CH_3$ | H | H | $-OCH_2-C(=O)-CH_3$ | H | | 142 |
| 20 | $-CH_2CH_2NEt_2$ | $CH_3$ | H | H | $-OCH_2CONMe_2$ | H | ·HCl | 206 |
| 21 | $-CH_2CH_2NEt_2$ | $CH_3$ | H | H | $OCH_2-C(=O)-NBu_2$ | H | ·HCl | 130 |
| 22 | $-CH_2CH_2NEt_2$ | $CH_3$ | H | H | $-OCH_2CON$ 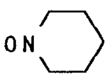 | H | | 121 |
| 23 | $-CH_2CH_2NEt_2$ | $CH_3$ | H | H | $-OCH_2-C(=O)-NHBu$ | H | | 129 |
| 24 | $-CH_2CH_2NEt_2$ | $CH_3$ | H | H | $-OCH_2-C(=O)-NHEt$ | H | | 125 |
| 25 | $-CH_2CH_2NEt_2$ | $CH_3$ | H | H | $-OCH_2CO_2iPr$ | H | ·HCl | 136 |
| 26 | $-CH_2CH_2NEt_2$ | $CH_3$ | H | H | $-OCH_2CO_2CH_2CH(CH_3)_2$ | H | ·HCl | 72 |
| 27 | $-CH_2CH_2NEt_2$ | $CH_3$ | H | H | $-OCH_2CO_2tert.Bu$ | H | ·HCl | 204 |

| Beispiel | $R^1$ | $R^2$ | $R^3$ | $R^4$ | $R^5$ | $R^6$ | Fp (°C) |
|---|---|---|---|---|---|---|---|
| 28 | $-CH_2CH_2NEt_2$ | $CH_3$ | H | H | $-OCH_2CONH(CH_2)_2NH_2$ | H | 116-118 |
| 29 | $-CH_2CH_2NEt_2$ | $CH_3$ | H | H | $-OCH_2CONH(CH_2)_2NH_2-\overset{O}{\overset{\parallel}{C}}-CH_3$ | H | 193 |
| 30 | $-CH_2CH_2NEt_2$ | $CH_3$ | H | H | $-OCH_2CONH(CH_2)_3NMe_2$ | H | 126 |
| 31 | $-(CH_2)_2\overset{\oplus}{N}Et_3\ Br^{\ominus}$ | $CH_3$ | H | H | $-OCH_2CO_2Et$ | | 92 |
| 32 | $-(CH_2)_2\overset{\oplus}{\underset{Me}{N}}Et_2\ J^{\ominus}$ | $CH_3$ | H | H | $-OCH_2CO_2Et$ | H | 109 |
| 33 | $-CH_2CO_2Et$ | $CH_3$ | H | H | $-OCH_2CH_2NEt_2$ | H ·HCl | 126-129 |
| 34 | $-CH_2CO_2Et$ | $CH_3$ | H | H | $-OCH_2CH_2NEt_2$ | $-OCH_2CH_2NEt_2$ | Öl |

Beispiel 35

$R^1$ = $-CH_2CO_2Et$ $R^2$ = $CH_3$ $R^3$ = $-OCH_2CH_2NEt_2$ $R^4$ = H $R^5$ = $-OCH_2CH_2NEt_2$ $R^6$ = H •2HCl Fp: 137 °C

Beispiel 36

$R^1$ = $(CH_2)_2NEt_2$ $R^2$ = $CH_3$ $R^3$ = H $R^4$ = H $R^5$ = OH

$R^6$ = 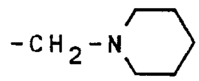 ·HCl

Fp. 254 °C

Beispiel 37

$R^1$ = $-(CH_2)_2NEt_2$ $R^2$ = $CH_3$ $R^3$ = H $R^4$ = H $R^5$ = $-OCH_2CO_2Et$

$R^6$ = 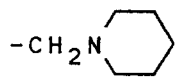 ·2HCl

Öl

Beispiel 38

$R^1$ = $-C_4H_9$ $R^2$ = $CH_3$ $R^3$ = H $R^4$ = H $R^5$ = $-OCH_2CO_2Et$

$R^6$ = 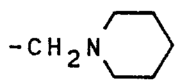 ·HCl

Fp. 117 °C

Beispiel 39

$R^1$ = 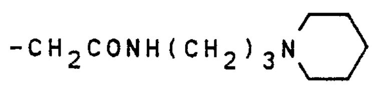

$R^2$ = Me $R^3$ = H $R^4$ = H $R^5$ = OH $R^6$ = H •HCl Fp. 255 °C

Beispiel 40

$R^1$ = $-CH_2CONH(CH_2)_2NEt_2$ $R^2$ = $CH_3$ $R^3$ = H $R^4$ = H $R^5$ = $-OCH_2CO_2Et$ $R^6$ = H •HCl Fp. 143 °C

Beispiel 41

$R^1$ = H $R^2$ = $-CH_2CONH(CH_2)_2NEt_2$ $R^3$ = H $R^4$ = H $R^5$ = OH $R^6$ = H Öl

Beispiel 42

$R^1$ = H

$R^2$ = 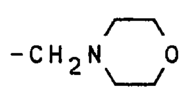

$R^3$ = H $R^4$ = H $R^5$ = $-OCH_2CO_2Et$ $R^6$ = H Fp.150 °C

Beispiel 43

$R^1$ = $-CH_2CH_2NEt_2$ $R^2$ = $CH_3$ $R^3$ = H $R^4$ = H $R^5$ = $-OCH_2CO_2Et$

$R^6$ = 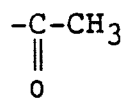 ·HCl

Fp.125 °C

Beispiel 44

$R^1$ = $-CH_2CH_2NEt_2$ $R^2$ = $CH_3$ $R^3$ = H $R^4$ = H $R^5$ = -OMe $R^6$ = H •HCl Fp.229 °C

Beispiel 45

$R^1$ = $-CH_2CH_2NEt_2$ $R^2$ = $CH_3$ $R^3$ = H $R^4$ = H

$R^5$ = 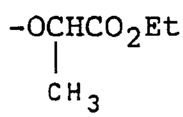

$R^6$ = H •HCl Fp.131 °C

Beispiel 46

$R^1$ = $-CH_2CH_2NEt_2$ $R^2$ = $CH_3$ $R^3$ = H $R^4$ = H $R^5$ = $-OCH_2CH_2CO_2Et$ $R^6$ = H •HCl Fp. 167 °C

Beispiel 47

$R^1$ = $-CH_2CH_2NEt_2$ $R^2$ = $CH_3$ $R^3$ = H $R^4$ = H $R^5$ = $-OCH_2CH_2CN$ $R^6$ = H •HCl Fp.210 °C

Beispiel 48

$R^1$ = $-CH_2CH_2NEt_2$ $R^2$ = $CH_3$ $R^3$ = H $R^4$ = H $R^5$ = $-OCH_2CONHCH_2CH_2OH$ $R^6$ = H •HCl Fp.168 °C

Beispiel 49

$R^1$ = $-CH_2CH_2NEt_2$ $R^2$ = $CH_3$ $R^3$ = H $R^4$ = H

$R^5$ = 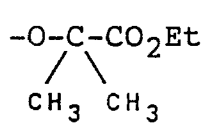

$R^6$ = H •HCl Fp.127 °C

Beispiel 50

$R^1$ = $-CH_2CH_2NEt_2$ $R^2$ = $CH_3$ $R^3$ = H $R^4$ = H $R^5$ = $-O(CH_2)_2NEt_2$ $R^6$ = H •2HCl Fp.254 °C

Beispiel 51

$R^1$ = $-CH_2CH_2NEt_2$ $R^2$ = $CH_3$ $R^3$ = H $R^4$ = H $R^5$ = $-OCH_2CO_2H$ $R^6$ = H

Beispiel 52

$R^1$ = $-CH_2CH_2NEt_2$ $R^2$ = $CH_3$ $R^3$ = H $R^4$ = H $R^5$ = $-OCH_2CO_2Et$ $R^6$ = $-NO_2$ •HCl Fp.198 °C

Beispiel 53

$R^1$ = $-CH_2CH_2NEt_2$ $R^2$ = $CH_3$ $R^3$ = H $R^4$ = H $R^5$ = $-OCH_2CO_2Et$ $R^6$ = $-NH_2$ •2HCl Fp.188 °C

Beispiel 54

$R^1$ = $-CH_2CH_2NEt_2$ $R^2$ = $CH_3$ $R^3$ = H $R^4$ = Cl $R^5$ = $-OCH_2CO_2Et$ $R^6$ = H •HCl Fp.211 °C

Beispiel 55

$R^1$ = H $R^2$ = 4-Pyridyl $R^3$ = H $R^4$ = H $R^5$ = $OCH_2CO_2Et$ $R^6$ = H •HCl Fp.206 °C

Beispiel 56

$R^1$ = H $R^2$ = 3-Pyridyl $R^3$ = H $R^4$ = H $R^5$ = $-OCH_2CO_2Et$ $R^6$ = H •HCl Fp:189 °C

Beispiel 57

$R^1$ = $-(CH_2)_2NEt_2$ $R^2$ = Me $R^3$ = $-OCH_2CONH_2$ $R^4$ = H $R^5$ = $-OCH_2CONH_2$ $R^6$ = H Fp.236 °C

Beispiel 58

$R^1$ = $-S-(CH_2)_2NEt_2$ $R^2$ = $CH_3$ $R^3$ = H $R^4$ = H $R^5$ = $-OCH_2CHCH_2$ $R^6$ = H •HCl Fp.153 °C

Beispiel 59

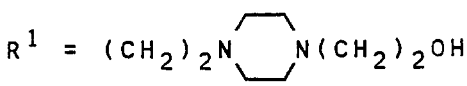

$R^2$ = $CH_3$ $R_3$ = H $R_4$ = H $R^5$ = $-OCH_2CO_2Et$ $R^6$ = H •2HCl Fp.212 °C

Beispiel 60

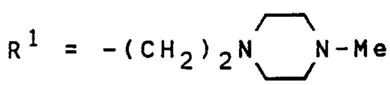

$R^2$ = $CH_3$ $R^3$ = H $R^4$ = H $R^5$ = $-OCH_2CO_2Et$ $R^6$ = H •2HCl Fp.241 °C

Beispiel 61

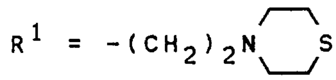

$R^2 = CH_3$ $R^3 = H$ $R^4 = H$ $R^5 = -OCH_2CO_2Et$ $R^6 = H$ •HCl Fp.226 °C

Beispiel 62

$R^1 = -(CH_2)_2NEt_2$ $R^2 = CH_3$ $R^3 = H$ $R^4 = -OCH_2CO_2Et$ $R^5 = -OCH_2CO_2Et$ $R^6 = H$ •HCl Fp.184 °C

Beispiel 63

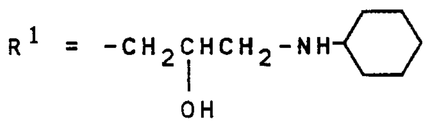

$R^2 = CH_3$ $R^3 = H$ $R^4 = H$ $R^5 = -OCH_2CO_2Et$ $R^6 = H$ •HCl Fp. 208-209 °C

Beispiel 64

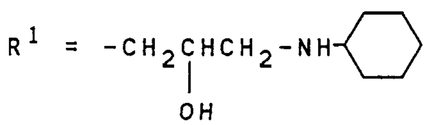

$R^2 = CH_3$ $R^3 = H$ $R^4 = H$ $R^5 = -HO$ $R^6 = H$ •HCl Fp.266 °C

Beipiel 65

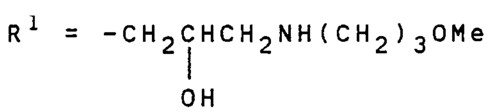

$R^2 = CH_3$ $R^3 = H$ $R^4 = H$ $R^5 = -OCH_2CO_2Et$ $R^6 = H$ •HCl Fp. 179 °C

Beispiel 66

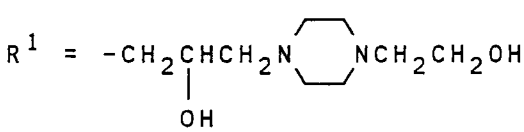

$R^2 = CH_3$ $R^3 = H$ $R^4 = H$ $R^5 = -OMe$ $R^6 = -OMe$ •2HCl Fp.240 °C

Beispiel 67

$R^1$ = H $R^2$ = Ph $R^3$ = H $R^4$ = H

$R^5$ = 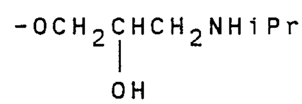

$R^6$ = H •HCl Fp.183-185°C

Beispiel 68

$R^1$ = Bu $R^2$ = Me $R^3$ = H $R^4$ = H

$R^5$ = 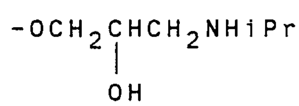

$R^6$ = H •HCl Fp.169-170°C

Beispiel 69

$R^1$ = $-CH_2CHCH_2$ $R^2$ = $CH_3$ $R^3$ = H $R^4$ = H

$R^5$ = 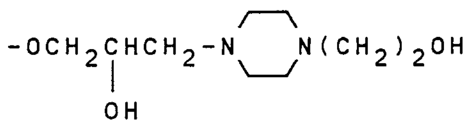

$R^6$ = H •2HCl Fp.222°C

Beispiel 70

$R^1$ = Bu $R^2$ = $-(CH_2)_2CH_3$ $R^3$ = H $R^4$ = H

$R^5$ = 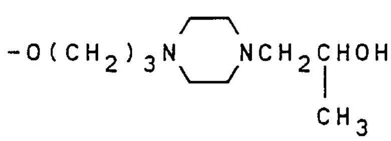

$R^6$ = H •2HCl Fp.215-217°C

Beispiel 71

$R^1$ = 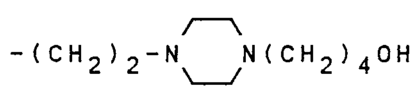

$R^2$ = $-(CH_2)_2CH_3$ $R^3$ = H $R^4$ = H $R^5$ = $OCH_3$ $R^6$ = $OCH_3$ •2HCl Fp. 200-203°C

Beispiel 72

$R^1$ = 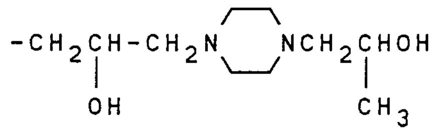

$R^2$ = Ph $R^3$ = H $R^4$ = $OCH_3$ $R^5$ = $OCH_3$ $R^6$ = H •2HCl Fp.224-225 °C

Beispiel 73

$R^1$ = 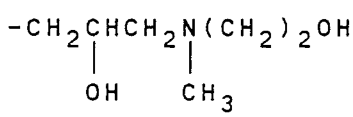

$R^2$ = $CH_3$ $R^3$ = OMe $R^4$ = H $R^5$ = OMe $R^6$ = H Fp.87 °C

Beispiel 74

$R^1$ = $-CH_2CONH(CH_2)_3NEt_2$ $R^2$ = $CH_3$ $R^3$ = H $R^4$ = OMe $R^5$ = OMe $R^6$ = H •2HCl Fp.208 °C

Beispiel 75

$R^1$ = $-(CH_2)_2NEt_2$ $R^2$ = $CH_3$ $R^3$ = H $R^4$ = H

$R^5$ = 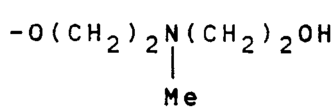

$R^6$ = H •2HCl Fp-195 °C

Beispiel 76

$R^1$ = 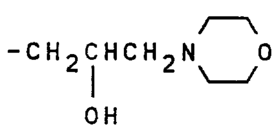

$R^2$ = $CH_3$ $R^3$ = OMe $R^4$ = OMe $R^5$ = OMe $R^6$ = H •HCl Fp.80 °C

Beispiel 77

$R^1$ = 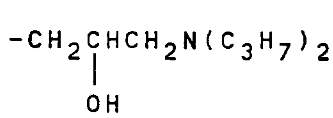

$R^2$ = Ph $R^3$ = H $R^4$ = Br $R^5$ = OMe $R^6$ = OMe •HCl Fp. 60 °C

Beispiel 78

$R^1 = -CH_2CH=CH_2$ $R^2 = CH_3$ $R^3 = H$ $R^4 = H$ $R^5 = OMe$

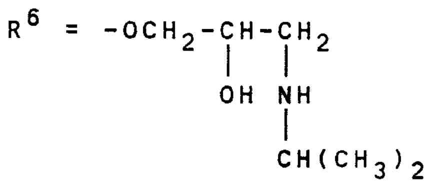

Fp.110 ° C

Beispiel 79

$R^1 = H$ $R^2 = CH_3$

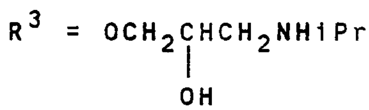

$R^4 = H$ $R^5 = H$ $R^6 = H$ • HCl Fp.205 ° C

Beispiel 80

$R^1 = -(CH_2)_2NEt_2$ $R^2 = CH_3$ $R^3 = H$ $R^4 = H$

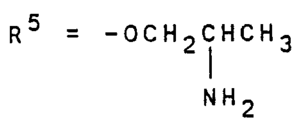

$R^6 = H$ • 2HCl Fp.188 ° C

Beispiel 81

$R^1 = -(CH_2)_2NEt_2$ $R^2 = CH_3$ $R^3 = H$ $R^4 = H$ $R^5 = OEt$

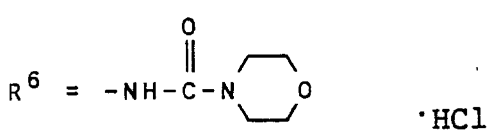

Fp.180 ° C

Beispiel 82

$R^1$ = $-(CH_2)_2NEt_2$ $R^2$ = $CH_3$ $R^3$ = H $R^4$ = H $R^5$ = $-NHCO_2Et$ $R^6$ = H •HCl Fp.244-246 °C

Beispiel 83

$R^1$ = $-(CH_2)_2NEt_2$ $R^2$ = $CH_3$ $R^3$ = H $R^4$ = H

$R^5$ = 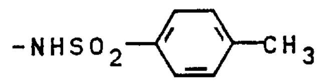

$R^6$ = H •HCl Fp.230 °C

Beispiel 84

$R^1$ = $-(CH_2)_2NEt_2$ $R^2$ = $CH_3$ $R^3$ = H $R^4$ = H

$R^5$ = 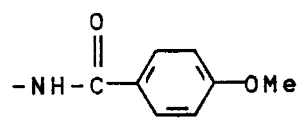

$R^6$ = H Fp.230 °C (Acetat)

Beispiel 85

$R^1$ = $-(CH_2)_2NEt_2$ $R^2$ = $CH_3$ $R^3$ = H $R^4$ = H $R^5$ = $-NHCH_2CO_2Et$ $R^6$ = H •HCl Fp.196 °C

Beispiel 86

$R^1$ = $-CH_2CONH(CH_2)_2NEt_2$ $R^2$ = $CH_3$ $R^3$ = H $R^4$ = H

$R^5$ = 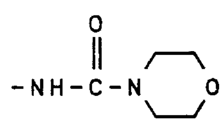

$R^6$ = H •HCl Fp.170 °C

Beispiel 87

$R^1$ = $-(CH_2)_2NEt_2$ $R^2$ = $CH_3$ $R^3$ = H $R^4$ = H

$R^5$ = 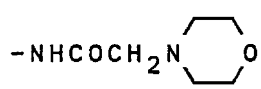 ·HCl

Fp.158-160 °C

Beispiel 88

$R^1$ = 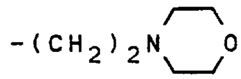

$R^2$ = $CH_3$ $R^3$ = H $R^4$ = H

$R^5$ = 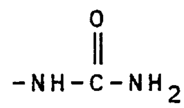

$R^6$ = H • HCl Fp.308-310 °C Zers.

Beispiel 89

$R^1$ = 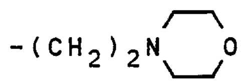

$R^2$ = $CH_3$ $R^3$ = H $R^4$ = H

$R^5$ = 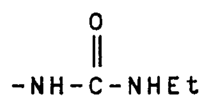

$R^6$ = H Fp.264-266 °C

Beispiel 90

$R^1$ = 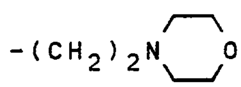

$R^2$ = $CH_3$ $R^3$ = H $R^4$ = H $R^5$ = -NHCONEt$_2$ $R^6$ = H Fp.215-217 °C

Beispiel 91

$R^1$ = 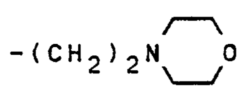

$R^2$ = $CH_3$ $R^3$ = H $R^4$ = H

$R^5$ = 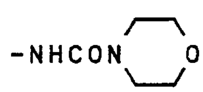

$R^6$ = H • HCl Fp.292-294 °C

Beispiel 92

$R^1$ = Pyrid-3-yl $R^2$ = H $R^3$ = H $R^4$ = H $R^5$ = O-$CH_2$-$CO_2Et$ $R^6$ = H Fp.: 158 - 161°C

Beispiel 93

$R^1$ = H $R^2$ = $CH_3$ $R^3$, $R^4$ = H $R^5$t= $NEt_2$ $R^6$ = H Fp.: 72 - 75°C

Beispiel 94

$R^1$ = $CH_2CH_2$-$NEt_2$ $R^2$ = $CH_3$ $R^3$, $R^4$ = H $R^5$ = NH-$CONHCO_2Et$ $R^6$ = H • HCl 193°C (Zers.)

**Patentansprüche**

**1.** Verwendung von Cumarinen der allgemeinen Formel I

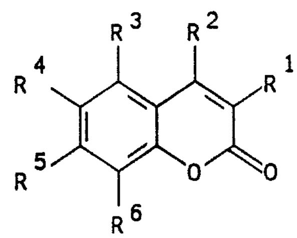

(I)

worin

**$R^1$** Wasserstoff; Halogen; ($C_2$-$C_6$)-Alkenyl; ($C_7$-$C_{10}$)-Aralkyl; Phenyl, das gegebenenfalls substituiert ist durch ($C_1$-$C_4$)-Alkyl, ($C_1$-$C_4$)-Alkoxy, Halogen, Nitro oder Amino; ($C_1$-$C_6$)-Alkyl, Hydroxy-($C_1$-$C_6$)-alkyl oder Dihydroxy-($C_1$-$C_6$)-alkyl, die gegebenenfalls durch ($C_1$-$C_4$)-Alkoxy, ($C_1$-$C_4$)-Alkoxycarbonyl, ($C_1$-$C_4$)-Alkylcarbonyl, Amino oder Aminocarbonyl, die auch N- oder Di-N-substituiert sein können,

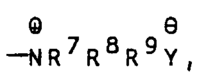 , 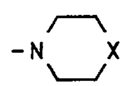 , 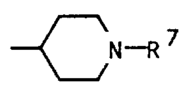 ,

Pyridyl oder Di-($C_1$-$C_4$)-alkylamino-($C_1$-$C_4$)-alkylthio substituiert sind; -CH($CH_2$-$NR^7R^8$)$_2$; oder Di-($C_1$-$C_4$)-alkylamino-($C_1$-$C_4$)-alkylthio bedeutet;

**$R^2$** ($C_1$-$C_6$)-Alkyl, das gegebenenfalls substituiert ist durch

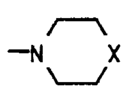

oder Amino oder Aminocarbonyl, die auch N- oder Di-N-substituiert sein können; ($C_2$-$C_6$)-Alkenyl; ($C_6$-$C_{10}$)-Aryl; oder Pyridyl bedeutet;

**$R^3$ und $R^4$** unabhängig voneinander Wasserstoff; Hydroxy; ($C_1$-$C_4$)-Alkoxy, Hydroxy-($C_1$-$C_4$)-alkoxy oder Dihydroxy-($C_1$-$C_4$)-alkoxy, die gegebenenfalls durch ($C_1$-$C_4$)-Alkoxycarbonyl, Amino oder Aminocarbonyl, die auch N- oder Di-N substituiert sein können, oder

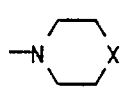

substituiert sind; ($C_2$-$C_6$)-Alkenyloxy; Amino-($C_1$-$C_4$)-alkyl, das auch N- oder Di-N substituiert sein kann;

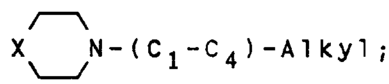

Halogen oder Nitro bedeuten;
**$R^5$** wie $R^3$ definiert ist und zusätzlich auch $CF_3$; $OR^7$; $(C_7\text{-}C_{10})$-Aralkoxy; $(C_1\text{-}C_4)$-Alkoxy, das durch ein oder zwei Hydroxygruppen oder

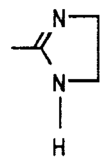

substituiert sein kann; $(C_1\text{-}C_6)$-Alkoxy, das durch $-COOR^7$ oder $-NR^7R^8$ substituiert ist; $(C_1\text{-}C_4)$-Alkylcarbonyl; $(C_2\text{-}C_6)$-Alkinyl; $(C_1\text{-}C_4)$-Alkoxycarbonyloxy; $NH_2$; oder $NHR^{10}$ bedeutet;
**$R^6$** wie $R^5$ definiert ist aber nicht für Chlor stehen kann,
**$R^7$** Wasserstoff; $(C_1\text{-}C_6)$-Alkyl, Hydroxy-$(C_1\text{-}C_6)$-alkyl oder Dihydroxy-$(C_1\text{-}C_6)$-alkyl, die gegebenenfalls durch Amino, Di-$(C_1\text{-}C_4)$-Alkylamino, $(C_1\text{-}C_4)$-Alkoxy, Cyan,

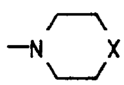

oder einen Rest $R^{10}$ substituiert sind; $(C_2\text{-}C_6)$-Alkenyl; oder $(C_7\text{-}C_{10})$-Aralkyl bedeutet;
**$R^8$** Wasserstoff; $(C_1\text{-}C_6)$-Alkyl; $(C_1\text{-}C_4)$-Alkyoxy-$(C_1\text{-}C_6)$-alkyl; oder $(C_7\text{-}C_{10})$-Aralkyl bedeutet;
**$R^9$** $(C_1\text{-}C_4)$-Alkyl bedeutet;
**$R^{10}$** Aminocarbonyl; N-$(C_1\text{-}C_4)$-Alkylaminocarbonyl; Di-N-$(C_1\text{-}C_4)$-Alkylaminocarbonyl; N-$(C_6\text{-}C_{10})$-Arylaminocarbonyl;

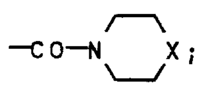

$(C_1\text{-}C_4)$-Alkylcarbonyl; $(C_6\text{-}C_{10})$-Arylcarbonyl; Di-N-$(C_1\text{-}C_4)$-Alkylaminomethylcarbonyl; $-SO_2(C_1\text{-}C_4)$-Alkyl oder $-SO_2(C_6\text{-}C_{10})$-Aryl bedeutet;
**X** eine Einfachbindung; O; $CH_2$; S; NH; N$(C_1\text{-}C_4)$-Alkyl; N$(C_6\text{-}C_{10})$-Aryl; oder N(Hydroxy-$(C_1\text{-}C_6)$-alkyl) bedeutet; und
**Y** Cl; Br; I; TosO; $HSO_4$ oder $CH_3COO$ bedeutet, sowie ihrer pharmakologisch annehmbaren Säureadditionsverbindungen zur Bekämpfung und Vorbeugung von Krankheiten, die durch einen erhöhten NO-Spiegel entstehen.

**2.** Verwendung gemäß Anspruch 1, dadurch gekennzeichnet, daß $R^1$ $(C_1\text{-}C_6)$-Alkyl, Di-$(C_1\text{-}C_4)$-alkylamino-$(C_1\text{-}C_6)$-alkyl,

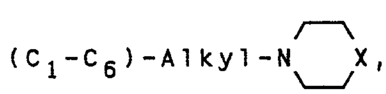

oder 3-Di-$(C_1\text{-}C_4)$-alkylamino-2-hydroxypropyl bedeutet.

**3.** Verwendung gemäß Anspruch 1 und/oder 2, dadurch gekennzeichnet, daß $R^2$ Methyl bedeutet.

**4.** Verwendung gemaß einem oder mehreren der Ansprüche 1 bis 3, dadurch gekennzeichnet, daß $R^3$ und $R^4$ unabhängig voneinander $(C_1\text{-}C_4)$-Alkoxy oder durch $NR^7R^8$ substituiertes Ethoxy bedeuten.

5. Verwendung gemaß einem oder mehreren der Ansprüche 1 bis 4, dadurch gekennzeichnet, daß $R^5$ Hydroxy, Methoxy, $(C_1\text{-}C_4)$-Alkoxy-carbonylmethoxy, Di-$(C_1\text{-}C_4)$-Dialkylaminocarbonylamino,

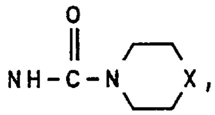

oder Amino-$(C_1\text{-}C_4)$-alkoxy bedeutet.

6. Verwendung gemaß einem oder mehreren der Ansprüche 1 bis 5, dadurch gekennzeichnet, daß $R^6$ Wasserstoff, Hydroxy, $(C_1\text{-}C_4)$-Alkoxy, Nitro oder Di-$(C_1\text{-}C_4)$-alkylamino-$(C_1\text{-}C_4)$-alkoxy bedeutet.

7. Verwendung gemäß einem oder mehreren der Ansprüche 1 bis 6 zur Bekämpfung und Vorbeugung pathologischer Blutdruckabfälle.

8. Verwendung gemaß einem oder mehreren der Ansprüche 1 bis 6 zur Bekämpfung und Vorbeugung von Colitis ulcerosa.

9. Verwendung gemäß einem oder mehreren der Ansprüche 1 bis 6 zur Bekämpfung und Vorbeugung des septischen Schocks.

10. Verwendung gemäß einem oder mehreren der Ansprüche 1 bis 6 zur Verhinderung von Transplantatabstoßungsreaktionen.

Europäisches Patentamt

# EUROPÄISCHER RECHERCHENBERICHT

Nummer der Anmeldung
EP 94 11 6789

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EUROPEAN J. PHARMACOLOGY,<br>Bd.243, Nr.2, 1993<br>Seiten 107 - 111<br>ZINGARELLI ET AL. 'Cloricromene inhibits the induction of nitric oxide synthase'<br>* siehe das ganze Dokument *<br>--- | 1-10 | A61K31/37 |
| X | DATABASE MEDLINE<br>DIALOG, file 155, accession no 05884770<br>* Zusammenfassung *<br>& ARCH INT. PHARMACODYN. THER.,<br>Bd.279, Nr.1, 1986<br>Seiten 103 - 120<br>FIEDLER VB. ET AL. 'Protective actions of carbocromene against amitriptyline-induced cardiotoxicity in anestized rats'<br>--- | 1-7 | |
| X | SURGERY GYNECOLOGY & OBSTETRICS,<br>Bd.143, Nr.4, Oktober 1976<br>Seiten 533 - 538<br>PEYTON M.D. ET AL., 'The effect of coronary vasodilatation on cardiac performance during endotoxin shock'<br>* siehe Seite 537 ("summary"), und Seite 535, linke Spalte, erster Abschnitt *<br>----- | 1-6,9 | RECHERCHIERTE SACHGEBIETE (Int.Cl.6)<br>A61K |

Der vorliegende Recherchenbericht wurde für alle Patentansprüche erstellt

| Recherchenort | Abschlußdatum der Recherche | Prüfer |
|---|---|---|
| MÜNCHEN | 23. Februar 1995 | Isert, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument